# EUROPÄISCHE PATENTSCHRIFT

(11) **EP 1 842 533 B1**
(45) Veröffentlichungstag und Bekanntmachung des Hinweises auf die Patenterteilung: **01.05.2013**
(21) Anmeldenummer: 07000943.6
(22) Anmeldetag: 05.08.2004
(51) Int. Cl.: A61K 9/20, A61K 31/515, A61K 31/485, A61K 31/5513, A61K 31/4725, A61K 9/16

(54) **Gegen Missbrauch gesicherte Darreichungsform**
Dosage form that is secured against misuse
Forme galénique empêchant un usage détourné

(30) Priorität: 06.08.2003 DE 10336400; 24.12.2003 DE 10361596; 22.04.2004 DE 102004020220; 01.07.2004 DE 102004032051
(43) Veröffentlichungstag der Anmeldung: 10.10.2007
(62) Teilanmeldung aus: 04763833.3
(73) Patentinhaber: Grünenthal GmbH, 52078 Aachen (DE)
(72) Erfinder: Bartholomäus, Johannes, Dr., 52080 Aachen (DE); Kugelmann, Heinrich, 52068 Aachen (DE); Arkenau-Maric, Elisabeth, Dr., 50931 Köln (DE)
(74) Vertreter: Kutzenberger, Helga

(56) Entgegenhaltungen:
- EP-A2- 1 138 321
- EP-A2- 1 166 776
- WO-A-01/52651
- US-A1- 2003 068 392

## Beschreibung

Die vorliegende Erfindung betrifft eine gegen Missbrauch gesicherte, ohne Verfärbung durch Extrusion thermogeformte Darreichungsform enthaltend neben einem oder mehreren Wirkstoffen mit Missbrauchspotential (A) sowie ggf. physiologisch verträglichen Hilfsstoffen (B) mindestens ein synthetisches oder natürliches Polymer (C) und ggf. mindestens ein Wachs (D), wobei die Komponente (C) sowie die gegebenenfalls vorhandene Komponente (D) jeweils eine Bruchfestigkeit von mindestens 500 N aufweist, sowie ein Verfahren zur Herstellung der erfindungsgemäßen Darreichungsform.

Eine Vielzahl von pharmazeutischen Wirkstoffen weist neben einer ausgezeichneten Wirksamkeit auf ihrem betreffenden Anwendungsgebiet auch ein Mißbrauchspotential auf, d.h. sie können von einem Mißbraucher eingesetzt werden, um Wirkungen herbeizuführen, die nicht ihrem Bestimmungszweck entsprechen.
So werden beispielsweise Opiate, die eine exzellente Wirksamkeit bei der Bekämpfung von starken bis sehr starken Schmerzen zeigen, von Mißbrauchem häufig zum Einleiten rauschartiger, euphorisierender Zustände verwendet.

Um Missbrauch zu ermöglichen, werden die entsprechenden Darreichungsformen wie Tabletten oder Kapseln vom Missbraucher zerkleinert, z. B. gemörsert, der Wirkstoff aus dem so erhaltenen Pulver mit Hilfe einer vorzugsweise wässrigen Flüssigkeit extrahiert und die resultierende Lösung, ggf. nach Filtration durch Watte oder Zellstoff, parenteral, insbesondere intravenös, appliziert. Bei dieser Art der Verabreichung kommt es zu einem gegenüber der oralen, missbräuchlichen Applikation noch zusätzlich beschleunigten Anfluten des Wirkstoffes mit dem vom Mißbraucher gewünschten Ergebnis, nämlich dem Kick. Dieser Kick wird auch erreicht, wenn die gepulverte Darreichungsform nasal appliziert, d. h. geschnupft wird. Da retardierte, orale Darreichungsformen, die Wirkstoffe mit Mißbrauchspotential enthalten, üblicherweise selbst bei einer oralen Einnahme von missbräuchlich hohen Mengen nicht zu dem vom Mißbraucher gewünschten Kick führen, werden auch diese zum Missbrauch zerkleinert und extrahiert.

Zur Verhinderung des Missbrauchs wurde in dem US-A- 4,070,494 vorgeschlagen, der Darreichungsform ein quellbares Mittel zuzusetzen. Dieses quillt bei der Zugabe von Wasser zur Extraktion des Wirkstoffes auf und bewirkt, dass das vom Gel separierte Filtrat nur eine geringe Menge an Wirkstoff enthält.

Ein entsprechender Ansatz zur Verhinderung des parenteralen Mißbrauchs liegt auch der in der WO 95/20947 offenbarten Mehrschichttablette zugrunde, die den Wirkstoff mit Mißbrauchspotential und mindestens einen Gelbildner jeweils in unterschiedlichen Schichten getrennt aufweist.

Ein weiterer Ansatz zur Verhinderung des parenteralen Mißbrauchs wird in der WO 03/015531 A2 offenbart. Dort wird eine Darreichungsform enthaltend ein analgetisches Opioid und einen Farbstoff als aversives Mittel beschrieben. Die Farbe, die durch unzulässige Manipulation der Darreichungsform freigesetzt wird, soll den Mißbraucher davon abhalten, diese manipulierte Darreichungsform zu verwenden.

Eine weitere bekannte Möglichkeit zur Erschwerung des Missbrauchs besteht darin, der Darreichungsform Antagonisten der Wirkstoffe, wie z. B. Naloxon oder Naltexon im Fall von Opioiden, oder Verbindungen, die zu physiologischen Abwehrreaktionen führen, wie z. B. Radix Ipecacuanha = Brechwurz, der Darreichungsform zuzusetzen.

Da aber nach wie vor in den meisten Fällen für den Missbrauch, eine Pulverisierung der Darreichungsformen mit einem zum Missbrauch geeigneten Wirkstoff notwendig ist, war es Aufgabe der vorliegenden Erfindung, die dem Missbrauch vorangehende Pulverisierung der Darreichungsform mit den einem potentiellen Missbraucher üblicherweise zur Verfügung stehenden Mitteln zu erschweren bzw. zu verhindern und somit eine feste Darreichungsform für Wirkstoffe mit Missbrauchspotential zur Verfügung zu stellen, die bei bestimmungsgemäßer Applikation die gewünschte therapeutische Wirkung gewährleistet, aus der aber die Wirkstoffe nicht durch einfaches Pulverisieren in eine zum Missbrauch geeignete Form übergeführt werden können.

Diese Aufgabe wurde durch die Bereitstellung der erfindungsgemäßen, gegen Missbrauch gesicherten, ohne Verfärbung durch Extrusion thermogeformten Darreichungsform, die neben einem oder mehreren Wirkstoffen mit Mißbrauchspotential (A) mindestens ein synthetisches oder natürliches Polymer (C) und ggf. mindestens ein Wachs (D) enthält, wobei die Komponente (C) und die gegebenenfalls vorhandene Komponente (D) jeweils eine Bruchfestigkeit von mindestens 500 N aufweist, gelöst.

Durch den Einsatz von Polymeren mit der angegebenen Mindestbruchfestigkeit (gemessen, wie in der Anmeldung angegeben), vorzugsweise in solchen Mengen, dass auch die Darreichungsform eine solche Mindestbruchfestigkeit von mindestens 500 N aufweist, gelingt es, ein Pulverisieren der Darreichungsform mit üblichen Mitteln zu verhindern und damit den anschließenden Missbrauch erheblich zu erschweren bzw. zu unterbinden.

Ohne ausreichende Zerkleinerung ist nämlich eine parenteral, insbesondere intravenöse, gefahrlose Applikation nicht möglich oder die Extraktion des Wirkstoffes daraus dauert für den Missbraucher zu lange bzw. ein Kick bei missbräuchlicher, oralen Einnahme erfolgt nicht, da keine spontane Freisetzung passiert.

Unter einer Zerkleinerung wird erfindungsgemäß die Pulverisierung der Darreichungsform mit üblichen Mitteln, die einem Missbraucher üblicherweise zur Verfügung stehen, wie z. B. ein Mörser und Pistill, ein Hammer, ein Schlegel oder andere gebräuchliche Mittel zum Pulverisieren unter Krafteinwirkung verstanden.

Die erfindungsgemäße Darreichungsform ist daher zur Verhinderung des parenteralen, nasalen und/oder oralen Missbrauchs von Wirkstoffen, vorzugsweise von pharmazeutischen Wirkstoffen, mit Mißbrauchspotential geeignet.

Pharmazeutische Wirkstoffe mit Mißbrauchspotential sind dem Fachmann ebenso wie deren einzusetzende Mengen und Verfahren zu deren Herstellung bekannt und können als solche, in Form ihrer dementsprechenden Derivate, insbesondere Ester oder Ether, oder jeweils in Form entsprechender physiologisch verträglicher Verbindungen, insbesondere in Form ihrer entsprechenden Salze oder Solvate, als Racemate oder Stereoisomere in der erfindungsgemäßen Darreichungsform vorliegen. Die erfindungsgemäße Darreichungsform eignet sich auch für die Verabreichung von mehreren pharmazeutischen Wirkstoffen in einer Darreichungsform. Vorzugsweise erhält die Darreichungsform nur einen bestimmten Wirkstoff.

Die erfindungsgemäße Darreichungsform eignet sich insbesondere zur Verhinderung des Missbrauchs wenigstens eines pharmazeutischen Wirkstoffs, der ausgewählt ist aus der Gruppe umfassend Opioide, Tranquillantien, vorzugsweise Benzodiazepine, Barbiturate, Stimulantien und weitere Betäubungsmittel.

Ganz besonders eignet sich die erfindungsgemäße Darreichungsform zur Verhinderung des Mißbrauchs eines Opioids, Tranquillanz oder eines anderen Betäubungsmittels, das ausgewählt ist aus der Gruppe umfassend N-{1-[2-(4-Ethyl-5-oxo-2-tetrazolin-1-yl)ethyl]-4-methoxymethyl-4-piperidyl}propionanilid (Alfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-α-Methylphenethylamin (Amfetamin), 2-(α-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3*H*)-on (Bromazepam), 2-Brom-4-(2-chlorphenyl)-9-methyl-6*H*-thieno[3,2-*f*][1,2,4]triazolo[4,3-a][1,4]diazepin (Brotizolam), 17-Cyclopropylmethyl-4,5α-epopxy-7α[(S)-1-hydroxy-1,2,2-trimethyl-propyl]-6-methoxy-6,14-*endo*-ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1*S*,2*S*)-2-Amino-1-phenyl-1-propanol (Cathin / D-Norpseudoephedrin), 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*)-dion (Clobazam), 5-(2-Chlorphenyl)-7-nitro-1*H*-1,4-benzodiazepin-2(3*H*)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H-*thieno[2,3-e][1,4]diazepin-2(3*H*)-on (Clotiazepam), 10-Chlor-11b-(2chlorphenyl)-2,3,7,11b-tetrahydrooxazolo[3,2-*d*][1,4]benzodiazepin-6(5*H*)-on (Cloxazolam), (-)-Methyl-[3β-benzoyloxy-2β(1αH,5α*H*)-tropancarboxylat] (Cocain), 4,5α-Epoxy-3-methoxy-17-methyl-7-morphinen-6α-ol (Codein), 5-(1-Cyclohexenyl)-5-ethylbarbitursäure (Cyclobarbital), Cyclorphan, Cyprenorphin, 7-Chlor-5-(2-chlorphenyl)-1*H*-1,4-benzodiazepin-2(3*H*)-on (Delorazepam), Desomorphin, Dextromoramid, (+)-(1-Benzyl-3-dimethylamino-2-methyl-1-phenylpropyl)propionat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimepheptanol, Dimethylthiambuten, Dioxaphetylbutyrat, Dipipanon, (6a*R*,10a*R*)-6,6,9-Trimethyl-3-pentyl-6a,7,8,10a-tetrahydro-6*H*-benzo[c]chromen-1-ol (Dronabinol), Eptazocin, 8-Chlor-6-phenyl-4H-[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethylloflazepat), 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-*enda*-etheno-morphinan-3-ol (Etorphin), *N-*Ethyl-3-phenyl-8,9,10-trinorboman-2-ylamin (Fencamfamin), 7-[2-(α-Methylphenethylamino)ethyl]-theophyllin) (Fenetyllin), 3-(α-Methylphenethylamino)propionitril (Fenproporex), N-(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1*H*-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1*H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3*S*,6*S*)-6-Dimethylamino-4,4-diphenylheptan-3-ylaretat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 6-(2-Chlorphenyl)-2-(4-methyl-l-piperazinylmethylen)-8-nitro-2*H-*imidazo[1,2-a][1,4] benzodiazepin-1(4*H*)-on (Loprazolam), 7-Chlor-5-(2-chlorphenyl)-3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H*-imidazo[2,1-*a*]isoindol-5-ol (Mazindol), 7-Chlor-2,3-dihydro-1-methyl-5-phenyl-1*H*-1,4-benzodiazepin (Medazepam), *N*-(3-Ch(orpropyl)-α-methylphenethylamin (Mefenorex), Meperidin, 2-Mothyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-*o*-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-*a*][1,4]benzodiazepin (Midazolam), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,10α-tetrahydro-1-hydroxy-6,6-dimethyl-6*H*-dibenzo [b, *d*]pyran-9(6α*H*)-on (Nabilon), Nalbuphen, Nalorphin, Narcein, Nicomorphin, 1-Methyl-7-nitro-5-phenyl-1*H*-1,4-benzodlazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyt-1*H*-1,4-benzodiazepin-2(3*H*)-on (Oxazepam), (*cis-trans*)-10-Chlor-2,3,7,11b-tetrahydro-2-methyl-11b-phenyloxazolo[3,2-*d*][1,4]benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pemolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), α ,α-Dimethylphenethylamin (Phentermin), 7-Chlor-5-phenyl-1-(2-propinyl)-1*H*-1 ,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-phenyl-1*H*-1,4-benzodiazepin-2(3*H*)-on (Prazepam), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), 5-sec-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3*H*)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chfor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1 R,2R)-3-(3-Dimethylamino-1-ethyl-2-methyl-propyl)-phenol, (1 R, 2R, 4S)-2-[Dimethylamino)methyl-4-(p-fluorbenzyloxy)-1-(m-methoxyphenyl)cyclohexanol, (1 R, 2R)-3-(2-Dimethylaminomethyl-cyclohexyl)-phenol, (1S, 2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1 RS, 3RS, 6RS)-6-Dimethylaminomethyl-1-(3-methoxy-phenyl)-cyclohexan-1,3-diol, vorzugsweise als Racemat, 3-(2-Dimethylaminomethyl-1-hydroxy-cyclohexyl)-phenyl 2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxynaphthalen-2-yl)-propionat, (RR-SS)-2-Acetoxy-4-trifluoromethyl-benzaesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxycyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt Hydrochloride.

Die erfindungsgemäße Darreichungsform eignet sich insbesondere zur Verhinderung des Missbrauchs eines opioiden Wirkstoffes ausgewählt aus der Gruppe umfassend Oxycodon, Hydromorphon, Morphin, Tramadol und deren physiologisch verträgliche Derivate oder Verbindungen, vorzugsweise deren Salze und Solvate, vorzugsweise deren Hydrochloride.

Weiterhin eignet sich die erfindungsgemäße Darreichungsform insbesondere zur Verhinderung des Missbrauchs eines opioiden Wirkstoffes ausgewählt aus der Gruppe umfassend (1 R, 2R)-3-(3-Dimethylamino-1-ethyl-2-methyl-propyl)-phenol, (2R, 3R)-1-Dimethylamino-3-(3-methoxy-phenyl)-2-methyl-pentan-3-ol, (1RS, 3RS, 6RS)-6-Dimethylaminomethyl-1-(3-methoxy-phenyl)-cyclohexane-1,3-diol, (1R, 2R)-3-(2-Dimethylaminonethyl-cyclohexyl)-phenol, deren physiologisch verträglichen Salze, vorzugsweise Hydrochloride, physiologisch verträgliche Enantiomere, Stereoisomere, Diastereomere und Racemate und deren physiologisch verträglichen Derivate, vorzugsweise Ether, Ester oder Amide.

Diese Verbindungen bzw. deren Herstellungsverfahren sind in der EP-A-693475 bzw. EP-A-780369 beschrieben.

Zur Erzielung der notwendigen Bruchfestigkeit der erfindungsgemäßen Darreichungsform werden mindestens ein synthetisches oder natürliches Polymer (C) mit einer Bruchfestigkeit, gemessen nach der in der vorliegenden Anmeldung offenbarten Methode, von mindestens 500 N eingesetzt. Bevorzugt wird hierfür mindestens ein Polymeres ausgewählt aus der Gruppe umfassend Polyalkylenoxide, vorzugsweise Polymethylenoxid, Polyethylenoxid, Polypropylenoxid; Polyethylen, Polypropylen, Polyvinylchlorid, Polycarbonat, Polystyrol, Polyacrylat, deren Copolymerisate und Mischungen aus mindestens zwei der genannten Polymeren eingesetzt. Bevorzugt sind hochmolekulare, thermoplastische Polyalkylenoxide. Besonders bevorzugt sind hochmolekulare Polyethylenoxide mit einem Molekulargewicht von mindestens 0,5 Mio., vorzugsweise mindestens 1 Mio. bis 15 Mio., bestimmt durch rheologische Messungen. Diese Polymeren weisen eine Viskosität bei 25 °C von 4500 bis 17600 cP, gemessen an einer 5 Gew.% wässrigen Lösung mit Hilfe eines Brookfield Viskosimeter, Model RVF (Spindel Nr. 2 /Rotationsgeschwindigkeit 2 rpm), von 400 bis 4000 cP, gemessen an einer 2 Gew.% wässrigen Lösung mit Hilfe des genannten Viskosimeters (Spindel Nr. 1 bzw. 3 /Rotationsgeschwindigkeit 10 rpm) bzw. von 1650 bis 10000 cP, gemessen an einer 1 Gew.% wässrigen Lösung mit Hilfe des genannten Viskosimeters (Spindel Nr. 2 /Rotationsgeschwindigkeit 2 rpm) auf.

Die Polymeren werden bevorzugt als Pulver eingesetzt. Sie können in Wasser löslich sein.

Des weiteren können zusätzlich zur Erzielung der notwendigen Bruchfestigkeit der erfindungsgemäßen Darreichungsform mindestens ein natürliches oder synthetisches Wachs (D) mit einer Bruchfestigkeit, gemessen nach der in der vorliegenden Anmeldung offenbarten Methode, von mindestens 500 N eingesetzt werden. Bevorzugt sind die Wachse mit einem Erweichungspunkt von mindestens 60°C. Besonders bevorzugt sind Camaubawachs und Bienenwachs. Ganz besonders bevorzugt ist Camaubawachs. Camaubawachs ist ein natürliches Wachs, das aus den Blättern der Carnaubapalme gewonnen wird und einen Erweichungspunkt von wenigstens ≥ 80°C aufweist. Beim zusätzlichen Einsatz der Wachskomponente wird diese zusammen mit wenigstens einem Polymeren (C) in solchen Mengen eingesetzt, dass die Darreichungsform eine Bruchfestigkeit von mindestens 500 N aufweist.

Vorzugsweise wird die Komponente (C) in einer Menge von 20 bis 99,9 Gew.%, besonders bevorzugt von wenigstens 30 Gew.%, ganz besonders bevorzugt von wenigstens 40 Gew.%, bezogen auf das Gesamtgewicht der Darreichungsform, eingesetzt.

Als Hilfsstoffe (B) können die üblichen für die Formulierung von festen Darreichungsformen bekannten Hilfsstoffe verwendet werden. Vorzugsweise sind dies Weichmacher, wie Polyethylenglykol, Hilfsstoffe, die Wirkstofffreisetzung beeinflussend, vorzugsweise hydrophobe oder hydrophile, vorzugsweise hydrophile Polymere, ganz besonders bevorzugt Hydroxypropylmethylcellulose oder Hydroxypropylcellulose, und/oder Antioxidantien. Als Antioxidantien eignen sich Ascorbinsäure, Butylhydroxyanisol, Butylhydroxytoluol, Salze der Ascorbinsäure, Monothioglyzerin, phosphorige Säure, Vitamin C, Vitamin E und dessen Derivate, Natriumbisulfit, besonders bevorzugt Butylhydroxytoluol (BHT) oder Butylhydroxyanisol (BHA) und α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Die erfindungsgemäßen Darreichungsformen zeichnen sich dadurch aus, dass sie aufgrund ihrer Härte mit Hilfe von üblichen, einem Missbraucher zur Verfügung stehenden Zerkleinerungsmitteln, wie Mörser und Pistill, nicht zu pulverisieren sind. Ein oraler, parenteraler, inbesondere intravenöser oder nasaler Missbrauch ist dadurch praktisch ausgeschlossen. Um jedoch jeden möglichen Missbrauch der erfindungsgemäßen Darreichungsformen vorzubeugen, können die erfindungsgemäßen Darreichungsformen in einer bevorzugten Ausführungsform als Hilfsstoffe (B) weitere Missbrauchs-erschwerende bzw, -verhindernde Mittel enthalten.

So kann die erfindungsgemäße, gegen Missbrauch gesicherte Darreichungsform, die neben einem oder mehreren Wirkstoffen mit Missbrauchspotential, mindestens einem härtebildenden Polymer (C) und ggf. mindestens einen Wachs (D) noch wenigstens eine der nachfolgenden Komponenten (a)-(e) als Hilfsstoffe (B) aufweist:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wässrigen Flüssigkeit aus der Darreichungsform gewonnenen Extrakt ein Gel bildet, welches vorzugsweise beim Einbringen in eine weitere Menge einer wässrigen Flüssigkeit visuell unterscheidbar bleibt,
(c) wenigstens einen Antagonisten für jeden der Wirkstoffe mit Missbrauchspotential,
(d) wenigstens ein Emetikum,
(e) wenigstens einen Farbstoff als aversives Mittel
(f) wenigstens einen Bitterstoff

Die Komponenten (a) bis (f) sind jeweils für sich allein zusätzlich zur Sicherung der erfindungsgemäßen Darreichungsform gegen Missbrauch geeignet. So eignet sich die Komponente (a) bevorzugt zur Sicherung gegen nasalen, oralen und/oder parenteralen, vorzugsweise intravenösen, Missbrauch, die Komponente (b) bevorzugt gegen parenteralen, besonders bevorzugt intravenösen und/oder nasalen Missbrauch, die Komponente (c) bevorzugt gegen nasalen und/oder parenteralen, besonders bevorzugt intravenösen, Missbrauch, die Komponente (d) vorzugsweise gegen parenteralen, besonders bevorzugt intravenösen, und/oder oralen und/oder nasalen Missbrauch, die Komponente (e) als visuelles Abschreckungsmittel gegen oralen oder parenteralen Missbrauch und die Komponente (f) gegen oralen oder nasalen Missbrauch. Durch die erfindungsgemäße Mitverwendung von wenigstens einer der vorstehend genannten Komponenten, gelingt es, bei erfindungsgemäßen Darreichungsformen noch effektiver den Missbrauch zu erschweren.

In einer Ausführungsform kann die erfindungsgemäße Darreichungsform auch zwei oder mehrere der Komponenten (a)-(f) in einer Kombination aufweisen, vorzugsweise (a), (b) und ggf. (c) und/oder (f) und/oder (e) bzw. (a), (b) und ggf. (d) und/oder (f) und/oder (e).

In einer weiteren Ausführungsform kann die erfindungsgemäße Darreichungsform sämtliche Komponenten (a)-(f) aufweisen.

Sofern die erfindungsgemäße Darreichungsform gegen Missbrauch die Komponente (a) umfasst, kommen als den Nasen- und/oder Rachenraum reizende Stoffe erfindungsgemäß sämtliche Stoffe in Betracht, die bei entsprechender Applikation über den Nasen- und/oder Rachenraum eine Reaktion des Körpers hervorrufen, die entweder für den Missbraucher so unangenehm ist, dass er die Applikation nicht weiter fortsetzen will oder kann, z.B. ein Brennen, oder die auf physiologische Art und Weise einer Aufnahme des entsprechenden Wirkstoffes entgegenwirken, z.B. über eine vermehrte nasale Sekretbildung oder Niesen. Diese üblicherweise den Nasen- und/oder Rachenraum reizenden Stoffe können auch bei parenteraler, insbesondere intravenöser, Applikation ein sehr unangenehmes Gefühl bis hin zu unerträglichen Schmerzen verursachen, so daß der Mißbraucher die Einnahme nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff.

Unter Darreichungseinheit wird eine separate bzw, separierbare Dosiseinheit, wie z. B. eine Tablette oder eine Kapsel, verstanden.

Vorzugsweise kann der erfindungsgemäßen Darreichungsform als Komponente (a) einer oder mehrere Inhaltsstoffe wenigstens einer Scharfstoffdroge, ausgewählt aus der Gruppe bestehend aus Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe bestehend aus Capsici Fructus (Paprika), Capsici Fructus acer (Cayennepfeffer) und Piperis nigri Fructus (Pfeffer), hinzugefügt werden.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist wenigstens ein Inhaltsstoff der Scharfstoffdrogen ausgewählt aus der Gruppe bestehend aus Myristicin, Elemicin, Isoeugenol, α -Asaron, Safrol, Gingerolen, Xanthorrhizol, Capsaicinoiden, vorzugsweise Capsaicin, Capsaicin- Derivate, wie N-vanillyl -9E-octadecenamid, Dihydrocapsaicin, Nordihydrocapsaicin, Homocapsaicin, Norcapsaicin, und Nomorcapsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und von diesen Inhaltsstoffen abgeleiteten Verbindungen.

Vorzugsweise kann die erfindungsgemäße Darreichungsform die Pflanzenteile der entsprechenden Scharfstoffdrogen in einer Menge von 0,01 bis 30 Gew.-%, besonders bevorzugt 0,1 bis 0,5 Gew.-%, jeweils bezogen auf das Gesamtgewicht der Darreichungseinheit, enthalten.
Kommen ein oder mehrere Inhaltsstoffe entsprechender Scharfstoffdrogen zum Einsatz, beträgt deren Menge in einer erfindungsgemäßen Darreichungseinheit bevorzugt 0,001 bis 0,005 Gew.-%, bezogen auf das Gesamtgewicht der Darreichungseinheit.

Eine weitere Möglichkeit bei der erfindungsgemäßen Darreichungsform gegen Missbrauch vorzubeugen, besteht darin, wenigstens ein viskositätserhöhendes Mittel als weitere Missbrauchs-verhindemde Komponente (b) der Darreichungsform zuzusetzen, das in einem mit Hilfe einer notwendigen Mindestmenge an einer wässrigen Flüssigkeit, vorzugsweise als ein aus der Darreichungsform gewonnenes wässriges Extrakt, ein Gel bildet, das kaum gefahrlos applizierbar ist und vorzugsweise beim Einbringen in eine weitere Menge einer wässrigen Flüssigkeit visuell unterscheidbar bleibt.

Visuelle Unterscheidbarkeit im Sinne der vorliegenden Erfindung bedeutet, dass das mit Hilfe einer notwendigen Mindestmenge an wässriger Flüssigkeit gebildete, Wirkstoff-haltige Gel beim Einbringen vorzugsweise mit Hilfe einer Injektionsnadel, in eine weitere Menge wäßriger Flüssigkeit von 37°C im wesentlichen unlöslich und zusammenhängend bleibt und nicht auf einfache Weise so dispergiert werden kann, dass eine parenterale, insbesondere intravenöse, gefahrlose Applikation möglich ist. - Vorzugsweise beträgt die Dauer der visuellen Unterscheidbarkeit wenigstens eine Minute, vorzugsweise mindestens 10 Minuten.

Die Viskositätserhöhung des Extrakts führt dazu, dass dessen Nadelgängigkeit bzw. Spritzbarkeit erschwert oder sogar unmöglich gemacht wird. Sofern das Gel visuell unterscheidbar bleibt, bedeutet dies, dass das erhaltene Gel beim Einbringen in eine weitere Menge wäßriger Flüssigkeit, z.B. durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, daß eine parenterale, insbesondere intravenöse, Applikation gefahrlos möglich ist. In Kombination mit mindestens einer ggf, vorhandenen Komponente (a) bis (e) führt dies zusätzlich zu unangenehmen Brennen, Erbrechen, schlechtem Geschmack und/oder zur visuellen Abschreckung.

Eine intravenöse Applikation eines entsprechenden Gels würde daher mit großer Wahrscheinlichkeit zur Verstopfung von Gefäßen, verbunden mit schweren gesundheitlichen Schäden des Missbrauchers führen.

Zur Überprüfung, ob ein viskositätserhöhendes Mittel als Komponente (b) zur Anwendung in der erfindungsgemäßen Darreichungsform geeignet ist, wird der Wirkstoff mit dem viskositätserhöhenden Mittel gemischt und in 10 ml Wasser bei einer Temperatur von 25 °C suspendiert. Bildet sich hierbei ein Gel, welches den obenstehend genannten Bedingungen genügt, eignet sich das entsprechende viskositätserhöhende Mittel zur zusätzlichen Missbrauchs-Vorbeugung bzw. - Verhinderung bei den erfindungsgemäßen Darreichungsformen.

Sofern der erfindungsgemäßen Darreichungsform die Komponente (b) hinzugefügt wird, kommen vorzugsweise eine oder mehrere viskositätserhöhende Mittel zum Einsatz, die ausgewählt sind aus der Gruppe umfassend mikrokristalline Cellulose mit 11 Gew.-% Carboxymethylcellulose-Natrium (Avicel^{®} RC 591), Carboxymethylcellulose-Natrium (Blanose^{®}, CMC-Na C300P^{®}, Frimulsion BLC-5^{®}, Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Pektine, vorzugsweise aus Citrusfrüchten oder Äpfeln (Cesapectin^{®} HM Medium Rapid Set), Wachsmaisstärke (C*Gel 04201^{®}), Natriumalginat (Frimulsion ALG (E401)^{®}), Guarkernmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150 ^{®}), Tarakemmehl (Polygum 43/1^{®}), Propylenglykolalginat (Protanal-Ester SD-LB^{®}), , Natrium-Hyaluronat, Tragant, Taragummi (Vidogum SP 200^{®}), fermentiertes Polysaccharid- Welan Gum (K1A96), Xanthan-Gummi (Xantural 180^{®}). Xanthane sind besonders bevorzugt. Die in Klammem angegebenen Bezeichnungen sind die Handelsnamen, unter denen die jeweiligen Materialien am Markt geführt sind. Im allgemeinen ist eine Menge von 0,1 bis 20 Gew.%, besonders bevorzugt 0,1 bis 15 Gew.%, bezogen auf das Gesamtgewicht der Darreichungsform, der/des genannten viskositätserhöhenden Mittels ausreichend, um die vorstehend genannten Bedingungen zu erfüllen.

Die viskositätserhöhenden Mittel der Komponente (b), sofern vorgesehen, liegen in der erfindungsgemäßen Darreichungsform bevorzugt in Mengen von ≥ 5 mg pro Darreichungseinheit, d.h. pro Dosiereinheit vor.

In einer besonders bevorzugten Ausführungsform der vorliegenden Erfindung kommen als Komponente (b) solche viskositätserhöhenden Mittel zum Einsatz, die bei der Extraktion aus der Darreichungsform mit der notwendigen Mindestmenge an wässriger Flüssigkeit ein Gel bilden, das Luftblasen einschließt. Die so erhaltenen Gele zeichnen sich durch ein trübes Erscheinungsbild aus, durch das der potentielle Missbraucher zusätzlich optisch gewarnt und von dessen parenteraler Applikation abgehalten wird.

Die Komponente (C) kann auch gegebenenfalls als zusätzliches viskositätserhöhendes Mittel dienen, das mit Hilfe einer notwendigen Mindestmenge einer wässrigen Flüssigkeit, ein Gel bildet.

Es ist auch möglich, die viskositätserhöhenden Mittel und die übrigen Bestandteile in räumlich voneinander getrennter Anordnung in der erfindungsgemäßen Darreichungsform zu formulieren.

Des weiteren kann die erfindungsgemäße Darreichungsform zur Vorbeugung und Sicherung gegen Missbrauch die Komponente (c) aufweisen, nämlich einen oder mehrere Antagonisten für den Wirkstoff bzw. die Wirkstoffe mit Missbrauchspotential, wobei die Antagonistenmenge vorzugsweise räumlich getrennt von den übrigen Bestandteilen der erfindungsgemäßen Darreichungsform vorliegen und keine Wirkung bei bestimmungsgemäßer Verwendung entfalten.

Geeignete Antagonisten zur Verhinderung des Mißbrauchs der Wirkstoffe sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Sofern der in der Darreichungsform vorliegende Wirkstoff ein Opioid ist, kommt als Antagonist bevorzugt ein Antagonist ausgewählt aus der Gruppe umfassend Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten, sofern eine Ausrüstung mit der Komponente (c) vorgesehen ist, in einer Menge von ≥ 1 mg, besonders bevorzugt in einer Menge von 3 bis 100 mg, ganz besonders bevorzugt in einer Menge von 5 bis 50 mg auf pro Darreichungsform, d.h. pro Dosiereinheit eingesetzt, Weist die erfindungsgemäße Darreichungsform als Wirkstoff ein Stimulanz auf, ist der Antagonist bevorzugt ein Neuroleptikum, vorzugsweise wenigstens eine Verbindung ausgewählt aus der Gruppe bestehend aus Haloperidol, Promethacin, Fluphenazin, Perphenazin, Levomepromazin, Thioridazin, Perazin, Chlorprothixin, Chlorprothixin, Zuclopentixol, Flupentixol, Prothipendyl, Zotepin, Benperidol, Pipamperon, Melperon und Bromperidol.

Vorzugsweise weist die erfindungsgemäße Darreichungsform diese Antagonisten in einer üblichen, dem Fachmann bekannten therapeutischen Dosierung, besonders bevorzugt in einer gegenüber der üblichen Dosierung verdoppelten bis verdreifachten Menge pro Dosiereinheit auf.

Sofern die Kombination zur Vorbeugung und Sicherung der erfindungsgemäßen Darreichungsform gegen Mißbrauch die Komponente (d) umfaßt, kann sie wenigstens ein Emetikum aufweisen, das vorzugsweise in einer räumlich getrennten Anordnung von den übrigen Komponenten der erfindungsgemäßen Darreichungsform vorliegen und bei bestimmungsgemäßer Anwendung keine Wirkung im Körper entfalten sollte.

Geeignete Emetika zur Verhinderung des Missbrauchs eines Wirkstoffs sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

In der erfindungsgemäßen Darreichungsform kann bevorzugt ein Emetikum auf Basis eines oder mehrerer Inhaltsstoffe von Radix Ipecacuanhae (Brechwurzel), vorzugsweise auf Basis des Inhaltsstoffes Emetin, in Betracht, wie sie z.B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden.

Vorzugsweise kann die erfindungsgemäße Darreichungsform als Komponente (d) das Emetikum Emetin aufweisen, bevorzugt in einer Menge von ≥ 3 mg, besonders bevorzugt ≥ 10 mg und ganz besonders bevorzugt in einer Menge von ≥ 20 mg pro Darreichungsform, d.h. Dosiereinheit.

Ebenfalls bevorzugt kann als Emetikum Apomorphin in der erfindungsgemäßen Missbrauchssicherung zum Einsatz kommen, vorzugsweise in einer Menge von vorzugsweise ≥ 3 mg, besonders bevorzugt ≥ 5 mg und ganz besonders bevorzugt ≥ 7 mg pro Dosiereinheit.

Sofern die erfindungsgemäße Darreichungsform die Komponente (e) als weiteren missbrauchsverhindernden Hilfsstoff enthält, so wird durch den Einsatz eines solchen Farbstoffes, insbesondere bei dem Versuch, den Wirkstoff für eine parenterale, vorzugsweise intravenöse Applikation, zu extrahieren, eine intensive Farbgebung einer entsprechenden wässrigen Lösung hervorgerufen, die zur Abschreckung beim potentiellen Missbraucher führen kann, Auch ein oraler Missbrauch, der üblicherweise über eine wässrige Extraktion des Wirkstoffes eingeleitet wird, kann durch diese Farbgebung verhindert werden. Geeignete Farbstoffe sowie die für die notwendige Abschreckungswirkung erforderlichen Mengen sind der WO 03/015531 zu entnehmen, wobei die entsprechende Offenbarung als Teil der vorliegenden Offenbarung gelten soll und hiermit als Referenz eingeführt wird.

Sofern die erfindungsgemäße Darreichungsform als zusätzlichen Missbrauchsverhindernden Hilfsstoff die Komponente (f) enthält, so wird durch diesen Zusatz von wenigstens einem Bitterstoff durch die damit eintretende Geschmacksverschlechterung der Darreichungsform der orale und/oder nasale Missbrauch zusätzlich verhindert.

Geeignete Bitterstoffe sowie die für den Einsatz wirksamen Mengen sind der US-2003/0064099 A1 zu entnehmen, deren entsprechende Offenbarung als Offenbarung der vorliegenden Anmeldung gelten soll und hiermit als Referenz eingeführt wird. Vorzugsweise eignen sich als Bitterstoffe Aromaöle, vorzugsweise Pfefferminzöl, Eukalyptusöl, Bittermandelöl, Menthol, Fruchtaromastoffe, vorzugsweise Aromastoffe von Zitronen, Orangen, Limonen, Grapefruit oder Mischungen davon, und/oder Denatonium-Benzoat (Bitrex®). Besonders bevorzugt ist Denatonium-Benzoat.

Die erfindungsgemäße feste Darreichungsform eignet sich zur oralen, vaginalen oder rektalen, vorzugsweise zur oralen Einnahme. Vorzugsweise ist sie nicht filmförmig. Die erfindungsgemäße Darreichungsform kann in multipartikulärer Form, bevorzugt in Form von Mikrotabletten, Mikrokapseln, Mikropellets, Granulaten, Sphäroiden, Perlen oder Pellets, ggf. in Kapseln abgefüllt oder zu Tabletten verpreßt, vorzugsweise zur oralen Verabreichung, vorliegen. Vorzugsweise weisen die multipartikulären Formen eine Größe bzw. Größenverteilung im Bereich von 0,1 bis 3 mm, besonders bevorzugt im Bereich von 0,5 bis 2 mm auf. Je nach gewünschter Darreichungsform werden ggf. auch die üblichen Hilfsstoffe (B) zur Formulierung der Darreichungsform mitverwendet.

Die erfindungsgemäß gegen Missbrauch gesicherte Darreichungsform wird durch Thermoverformung mit Hilfe eines Extruders hergestellt, ohne dass dabei eine Verfärbung des Extrudates zu beobachten ist.

Um das Ausmaß der Verfärbung durch diese Thermoformgebung zu untersuchen, wird zunächst die Färbung der Mischung der Ausgangkomponenten, aus denen die Darrreichungsform besteht, ohne Zugabe einer farbgebenden Komponente, wie z. B. einem Farbpigment oder einer eigengefärbten Komponente (z. B. α-Tocopherol) festgestellt. Diese Zusammensetzung wird dann erfindungsgemäß thermogeformt,
wobei sämtliche Verfahrensschritte einschließlich der Kühlung des Extrudates unter einer Inertgasatmosphäre durchgeführt werden. Im Vergleich dazu wird dieselbe Zusammensetzung nach demselben Verfahren aber ohne Inertgasatmosphäre hergestellt. Von der Ausgangszusammensetzung der erfindungsgemäß hergestellten Darreichungsform und der zum Vergleich hergestellten Darreichungsform wird die Färbung bestimmt. Die Bestimmung erfolgt mit Hilfe von "Munsell Book of Colour" von Munsell Colour Company Baltimore, Maryland, USA, Ausgabe 1966. Sofern die Färbung der erfindungsgemäß thermogeformten Darreichungsform eine Färbung mit der Identifikations-Nr. N 9,5/ höchstens jedoch eine Färbung mit der Identifikations-Nr. 5Y 9/1 aufweist, wird die Thermoverformung als eine "ohne Verfärbung" eingestuft. Sofern die Darreichungsform eine Färbung mit der identifikations-Nr. 5Y 9/2 oder mehr aufweist bestimmt gemäß Munsell Book of Colour wird die Thermoverformung als eine "mit Verfärbung" eingestuft.

Überraschenderweise weisen die erfindungsgemäß gegen Missbrauch gesicherten festen Darreichungsformen keine gemäß der vorstehenden Klassifizierung einzustufende Verfärbung auf, wenn das gesamte Herstellungsverfahren unter einer Inertgasatmosphäre, vorzugsweise unter einer Stickstoffatmosphäre mit Hilfe eines Extruders zur Thermoformgebung durchgeführt wird.

Ein weiterer Gegenstand der vorliegenden Erfindung ist daher ein Verfahren zur Herstellung der erfindungsgemäßen gegen Missbrauch gesicherten Darreichungsformen, das dadurch gekennzeichnet ist, dass man
z) die Komponenten (A), (B), (C) und die gegebenenfalls vorhandene Komponente (D) mischt sowie die gegebenenfalls vorhandenen Komponenten a) bis f) mitmischt oder, soweit notwendig, separat unter Einsatz der Komponente (C) und gegebenenfalls (D) mischt,
y) die resultierende Mischung oder die resultierenden Mischungen im Extruder mindestens bis zum Erweichungspunkt der Komponente (C) erwärmt und unter Krafteinwirkung durch die Austrittsöffnung des Extruders extrudiert,
x) das noch plastische Extrudat vereinzelt und zur Darreichungsform formt oder
w) das abgekühlte und ggf. wieder erwärmte vereinzelte Extrudat zur Darreichungsform formt,
wobei die Verfahrensschritte y) und x) und ggf. die Verfahrensschritte z) und w) unter Inertgasatmosphäre, vorzugsweise Stickstoffatmosphäre, durchgeführt werden.

Das Mischen der Komponenten gemäß dem Verfahrensschritt z) kann ebenfalls bereits in dem Extruder erfolgen.

Die Mischung der Komponenten (A), (B), (C) und ggf. (D) sowie bzw. der ggf. vorhandenen weiteren Komponenten (a) - (f) und ggf, der Komponenten (C) und der ggf. vorhandenen Komponente (D) kann auch ggf. jeweils in einem dem Fachmann bekannten Mischgerät erfolgen. Das Mischgerät kann beispielsweise ein Wälzmischer, Schüttelmischer, Schermischer oder Zwangsmischer sein.

Vorzugsweise wird vor dem Abmischen mit den weiteren Komponenten die Komponente (C) und die gegebenenfalls vorhandene Komponente (D) erfindungsgemäß mit einem Antioxidanz versehen. Dies kann durch Vermischen der beiden Komponenten (C) und dem Antioxidanz erfolgen, vorzugsweise indem das Antioxidant in einem leicht flüchtigem Lösungsmittel aufgelöst oder suspendiert und diese Lösung oder Suspension mit der Komponente (C) und der gegebenenfalls vorhandenen Komponente (D) homogen vermischt und das Lösungsmittel durch Trocknung, vorzugsweise unter Inertgasatmosphäre, entfernt wird.

Zur Herstellung der erfindungsgemäßen Darreichungsformen, die Untereinheiten mit weiteren missbrauchsverhindemden bzw. erschwerenden Hilfsstoffen enthalten, können die Mischungen gemäß z) coextrudiert oder separat extrudiert werden.

Auf jeden Fall wird die im Extruder bis mindestens zum Erweichungspunkt der Komponente (C) erwärmte, vorzugsweise schmelzflüssige Mischung bzw. Mischungen aus dem Extruder durch eine Düse mit mindestens einer Bohrung extrudiert.

Vorzugsweise werden zur Durchführung des erfindungsgemäßen Verfahrens übliche Extruder, besonders bevorzugt Schneckenextruder, die sowohl mit einer oder mit zwei Schnecken ausgerüstet sind, eingesetzt.

Vorzugsweise weist der Extruder mindestens zwei Temperaturzonen auf, wobei in der ersten Zone, die sich an eine Einzugs- und ggf. Mischzone anschließt, das Aufheizen der Mischung bis mindestens zum Erweichungspunkt der Komponente (C) stattfindet. Der Durchsatz der Mischung liegt vorzugsweise bei 2,0 kg bis 8,0 kg/Stunde.

Nach Erwärmung bis mindestens zum Erweichungspunkt der Komponente (C) wird die aufgeschmolzene Mischung mit Hilfe der Schnecken gefördert, weiter homogenisiert, komprimiert bzw. kompaktiert, so dass sie unmittelbar vor dem Austritt aus der Extruderdüse einen Mindestdruck von 5 bar, vorzugsweise mindestens 10 bar aufweist, und durch die Düse als Extrusionsstrang oder Extrusionsstränge je nach dem wie viele Bohrungen die Düse aufweist, extrudiert. Die Düsengeometrie bzw. die Geometrie der Bohrungen ist frei wählbar. So kann die Düse bzw. die Bohrungen einen runden, oblongen oder ovalen Querschnitt aufweisen, wobei der runde Querschnitt vorzugsweise einen Durchmesser 0,1 mm bis 15 mm, der oblonge Querschnitt vorzugsweise mit einer maximalen Längsanpassung von 21 mm und einer Querausdehnung von 10 mm vorliegt. Vorzugsweise hat die Düse bzw. die Bohrungen einen runden Querschnitt. Der Mantel des erfindungsgemäß zum Einsatz kommenden Extruders kann beheizt oder gekühlt werden. Die entsprechende Temperierung, d. h. Beheizung oder Kühlung, richtet sich danach, dass die zu extrudierende Mischung mindestens eine Durchschnittstemperatur (Produkttemperatur) entsprechend der Erweichungstemperatur der Komponente (C) aufweist und nicht über eine Temperatur steigt, bei der der zur verarbeitende Wirkstoff mit Missbrauchspotential Schaden nehmen kann. Vorzugsweise wird die Temperatur der zu extrudierenden Mischung unter 180 °C, vorzugsweise unter 150 °C, aber mindestens auf die Erweichungstemperatur der Komponente (C), eingestellt.

Nach der Extrusion der geschmolzenen Mischung und gegebenenfalls Kühlung des extrudierten Stranges bzw. der extrudierten Stränge erfolgt vorzugsweise eine Zerkleinerung der Extrudate. Diese Zerkleinerung kann vorzugsweise durch Zerschneiden der Extrudate mittels mitlaufender oder rotierender Messer, Wasserstrahlschneider, Drähten, Klingen oder mit Hilfe von Laserschneidern durchgeführt werden.

Für eine Zwischenlagerung bzw. Endlagerung des ggf. vereinzelten Extrudates bzw. der Endform der erfindungsgemäßen Darreichungsform ist keine Inertgasatmosphäre notwendig.

Das vereinzelte Extrudat kann mit üblichen Methoden pelletisiert oder zu Tabletten verpresst werden, um der Darreichungsform die Endform zu geben. Es ist aber auch möglich, das strangförmige Extrudat nicht zu vereinzeln und mit Hilfe von gegenläufigen Kalanderwalzen, die auf ihren Umlaufmantel gegenüberliegende Vertiefungen aufweisen, zur Endform, vorzugsweise zu einer Tablette, zu formen und diese mit Hilfe üblicher Methoden zu vereinzeln.

Sollte das gegebenenfalls vereinzelte Extrudat nicht sofort zur Endform geformt, sondern zur Lagerung abgekühlt werden, so ist nach der Lagerung für eine Inertgasatmosphäre, vorzugsweise für eine Stickstoffatmosphäre zu sorgen, die bei einem Erwärmen des gelagerten Extrudats bis zur Plastifizierung und endgültigen Formgebung zur Darreichungsform eingehalten werden muss.

Die Krafteinwirkung im Extruder auf die zumindest plastifizierte Mischung wird durch Steuerung der Umdrehungsgeschwindigkeit der Fördereinrichtung im Extruder und deren Geometrie und durch die Dimensionierung der Austrittsöffnung so eingestellt, dass sich im Extruder vorzugsweise vor der unmittelbaren Extrudierung der plastifizierten Mischung der dafür notwendige Druck aufbaut. Durch einfache Vorversuche kann für die jeweilige Zusammensetzung die notwendigen Extrusionsparameter festgestellt werden, die zu einer Darreichungsform mit einer Bruchfestigkeit von mindestens 500 N führt.

In einer weiteren bevorzugten Ausführungsform liegt die erfindungsgemäße Darreichungsform in Form einer Tablette, einer Kapsel oder in Form eines oralen osmotischen therapeutischen Systems (OROS) vor, vorzugsweise wenn mindestens noch eine weitere missbrauchsverhindernde Komponente (a)-(f) vorhanden ist.

Sofern die Komponenten (c) und/oder (d) und/oder (f) in der erfindungsgemäßen Darreichungsform vorhanden sind, ist darauf zu achten, dass sie so formuliert oder so gering dosiert sind, daß sie bei bestimmungsgemäßer Applikation der Darreichungsform praktisch keine den Patienten oder die Wirksamkeit des Wirkstoffs beeinträchtigende Wirkung entfalten können.

Wenn die erfindungsgemäße Darreichungsform die Komponente (d) und/oder (f) enthält, ist die Dosierung so zu wählen, dass bei bestimmungsgemäßer oraler Applikation keine negative Wirkung hervorgerufen wird. Wird jedoch die vorgesehene Dosierung bei einem Missbrauch überschritten, wird Übelkeit bzw. Brechreiz bzw. schlechter Geschmack hervorgerufen. Die jeweilige Menge der Komponente (d) und/oder (f), die vom Patienten bei bestimmungsgemäßer oraler Applikation noch toleriert wird, kann vom Fachmann durch einfache Vorversuche ermittelt werden.

Sofern aber unabhängig von der praktisch nicht möglichen Pulverisierbarkeit der erfindungsgemäßen Darreichungsform zur Sicherung der Darreichungsform der Einsatz der Komponenten (c) und/oder (d) und/oder (f) vorgesehen ist, sollten diese Komponenten bevorzugt in einer so hohen Dosierung zum Einsatz kommen, dass sie bei einer missbräuchlichen Applikation der Darreichungsform eine intensive negative Wirkung beim Missbraucher hervorrufen. Dies gelingt vorzugsweise durch eine räumliche Trennung zumindest des Wirkstoffes bzw. der Wirkstoffe von den Komponenten (c) und/oder (d) und/oder (f), wobei bevorzugt der Wirkstoff bzw. die Wirkstoffe in wenigstens einer Untereinheit (X) und die Komponenten (c) und/oder (d) und/oder (f) in wenigstens einer Untereinheit (Y) vorliegen, und wobei die Komponenten (c), (d) und (f) bei bestimmungsgemäßer Applikation der Darreichungsform bei Einnahme und/oder im Körper nicht ihre Wirkung entfalten und die übrigen Formulierungskomponenten insbesondere die Komponente (C) und ggf. (D) identisch sind.

Sofern die erfindungsgemäße Darreichungsform wenigstens 2 der Komponenten (c) und (d) bzw. (f) aufweist, können diese jeweils in derselben oder in verschiedenen Untereinheiten (Y) vorliegen. Vorzugsweise liegen, sofern vorhanden, alle Komponenten (c) und (d) und (f) in ein- und derselben Untereinheit (Y) vor.

Untereinheiten im Sinne der vorliegenden Erfindung sind feste Formulierungen, die jeweils neben üblichen, dem Fachmann bekannten Hilfsstoffen den (die) Wirkstoff(e), mindestens ein Polymer (C) und die gegebenenfalls vorhandene Komponente (D) und gegebenenfalls wenigstens eine der gegebenenfalls vorhandenen Komponenten (a) und/oder (b) und/oder (e) bzw. jeweils wenigstens ein Polymer (C) und gegebenenfalls (D) und den (die) Antagonist(en) und/oder das Emetikum (die Emetika) und/oder die Komponente (e) und/oder die Komponente (f) und gegebenenfalls wenigstens eine der gegebenenfalls vorhandenen Komponenten (a) und/oder (b) enthalten. Dabei ist darauf zu achten, dass jede der genannten Untereinheiten nach den vorstehend angegebenen Verfahren formuliert werden.

Ein wesentlicher Vorteil der getrennten Formulierung der Wirkstoffe von den Komponenten (c) bzw. (d) bzw. (f) in Untereinheiten (X) und (Y) der erfindungsgemäßen Darreichungsform besteht darin, dass bei ihrer bestimmungsgemäßen Applikation die Komponenten (c) und/oder (d) und/oder (f) bei Einnahme und/oder im Körper praktisch nicht freigesetzt werden oder nur in so geringen Mengen freigesetzt werden, dass sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist. Vorzugsweise werden die Komponenten (c) und/oder (d) und/oder (f) bei bestimmungsgemäßer Applikation der Darreichungsform im Körper des Patienten praktisch nicht freigesetzt oder vom Patienten nicht wahrgenommen.

Der Fachmann versteht, dass diese vorstehend genannten Bedingungen in Abhängigkeit von den jeweils eingesetzten Komponenten (c), (d) und/oder (f) sowie der Formulierung der Untereinheiten bzw. der Darreichungsform variieren können. Die für die jeweilige Darreichungsform optimale Formulierung kann durch einfache Vorversuche ermittelt werden. Entscheidend ist, dass die jeweiligen Untereinheiten das Polymer (C) und gegebenenfalls die Komponente (D) enthalten und in der vorstehend angegebenen Weise formuliert wurden.

Sollte es den Missbrauchern wider Erwarten gelingen, eine solche erfindungsgemäße Darreichungsform, welche die Komponenten (c) und/oder (e) und/oder (d) und/oder (f) in Untereinheiten (Y) aufweist, zum Zwecke der mißbräuchlichen Einnahme des Wirkstoffes zu zerkleinern und ein Pulver zu erhalten, das mit einem geeigneten Extraktionsmittel extrahiert wird, wird neben dem Wirkstoff auch die jeweilige Komponente (c) und/oder (e) und/oder (f) und/oder (d) in einer Form erhalten, in der sie von dem Wirkstoff nicht auf einfache Weise zu separieren ist, so dass sie bei der Applikation der manipulierten Darreichungsform, insbesondere bei oraler und/oder parenteraler Verabreichung, ihre Wirkung bei Einnahme und/oder im Körper entfaltet und zusätzlich eine der Komponente (c) und/oder (d) und/oder (f) entsprechende negative Wirkung beim Missbraucher hervorruft oder ein Versuch, den Wirkstoff zu extrahieren durch die Farbgebung abschreckt und so den Missbrauch der Darreichungsform verhindert.

Die Formulierung einer erfindungsgemäßen Darreichungsform, in der eine räumliche Trennung des Wirkstoffes bzw. der Wirkstoffe von den Komponenten (c), (d) und/oder (e), vorzugsweise durch Formulierung in verschiedenen Untereinheiten erfolgt ist, kann in vielfältiger Art und Weise erfolgen, wobei die entsprechenden Untereinheiten in der erfindungsgemäßen Darreichungsform jeweils in beliebiger räumlicher Anordnung zueinander vorliegen können, sofern die vorstehend genannten Bedingungen für die Freisetzung der Komponenten (c) und/oder (d) erfüllt sind.

Der Fachmann versteht, dass die ggf. auch vorliegenden Komponente(n) (a) und/oder (b) bevorzugt sowohl in den jeweiligen Untereinheiten (X) und (Y) als auch in Form von eigenständigen, den Untereinheiten (X) und (Y) entsprechenden Untereinheiten in der erfindungsgemäßen Darreichungsform formuliert werden können, so lange die Sicherung der Darreichungsform gegen den Missbrauch wie auch die Wirkstofffreisetzung bei bestimmungsgemäßer Applikation durch die Art der Formulierung nicht beeinträchtig werden und das Polymer (C) und gegebenenfalls (D) mit formuliert und die Formulierung gemäß den vorstehend angegebenen Verfahren zur Erzielung der notwendigen Härte durchgeführt wird.

In einer bevorzugten Ausführungsform der erfindungsgemäßen Darreichungsform liegen die Untereinheiten (X) und (Y) in multipartikulärer Form vor, wobei Mikrotabletten, Mikrokapseln, Mikropellets, Granulaten, Sphäroiden, Perlen oder Pellets bevorzugt sind und sowohl für die Untereinheit (X) als auch (Y) dieselbe Form, d.h. Gestaltung gewählt wird, damit keine Separierung der Untereinheiten (X) von (Y), z. B. durch mechanische Auslese, möglich ist. Die multipartikulären Formen weisen bevorzugt eine Größe im Bereich von 0,1 bis 3 mm, vorzugsweise 0,5 bis 2 mm auf.

Die Untereinheiten (X) und (Y) in multpartikulärer Form können auch bevorzugt in eine Kapsel abgefüllt oder zu einer Tablette verpreßt werden, wobei die jeweiligen Endformulierungen dergestalt erfolgen, dass die Untereinheiten (X) und (Y) auch in der resultierenden Darreichungsform erhalten bleiben.

Die jeweiligen multipartikulären Untereinheiten (X) bzw. (Y) mit identischer Formgebung sollten auch nicht visuell voneinander unterscheidbar sein, damit sie vom Missbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z.B. die Retardierung eines oder mehrerer Wirkstoffe oder eine magensaftresistente Ausrüstung der jeweiligen Untereinheiten.

Die multipartikulären Untereinheiten können auch als Slurry oder als Suspension in pharmazeutisch unbedenklichen Suspensionsmedien als orale Darreichungsform formuliert werden.

In einer weiteren bevorzugten Ausführungsform der vorliegenden Erfindung sind die Untereinheiten (X) und (Y) jeweils schichtförmig zueinander angeordnet.

Bevorzugt sind hierfür die schichtförmigen Untereinheiten (X) und (Y) in der erfindungsgemäßen Darreichungsform vertikal oder horizontal zueinander angeordnet, wobei jeweils auch eine oder mehrere schichtförmige Untereinheiten (X) und eine oder mehrere schichtförmige Untereinheiten (Y) in der Darreichungsform vorliegen können, so daß neben den bevorzugten Schichtenfolgen (X)-(Y) bzw. (X)-(Y)-(X) beliebige andere Schichtenfolgen in Betracht kommen, ggf. in Kombination mit Schichten enthaltend die Komponenten (a) und/oder (b).

Ebenfalls bevorzugt ist eine erfindungsgemäße Darreichungsform, in der die Untereinheit (Y) einen Kern bildet, der von der Untereinheit (X) vollständig umhüllt wird, wobei zwischen diesen Schichten eine Trennschicht (Z) vorhanden sein kann. Ein entsprechender Aufbau eignet sich bevorzugt auch für die vorstehend genannten multipartikulären Formen, wobei dann beide Untereinheiten (X) und (Y) sowie eine ggf. vorhandene Trennschicht (Z), die der erfindungsgemäßen Härteanforderung genügen muss, in ein- und derselben multipartikulären Form formuliert sind. In einer weiteren bevorzugten Ausführungsform der erfindungsgemäßen Darreichungsform bildet die Untereinheit (X) einen Kern, der von der Untereinheit (Y) umhüllt wird, wobei letztere wenigstens einen Kanal aufweist, der von dem Kern an die Oberfläche der Darreichungsform führt.

Zwischen einer Schicht der Untereinheit (X) und einer Schicht der Untereinheit (Y) kann die erfindungsgemäße Darreichungsform jeweils eine oder mehrere, vorzugsweise eine, ggf. quellbare Trennschicht (Z) zur räumlichen Trennung der Untereinheit (X) von (Y) aufweisen.

Sofern die erfindungsgemäße Darreichungsform die schichtförmigen Untereinheiten (X) und (Y) sowie eine ggf. vorhandene Trennschicht (Z) in einer zumindest teilweise vertikalen oder horizontalen Anordnung aufweist, liegt sie bevorzugt in Form einer Tablette oder eines Laminats vor.

Hierbei kann in einer besonders bevorzugten Ausführungsform die freie Oberfläche der Untereinheit (Y) vollständig und ggf. zumindest ein Teil der freien Oberfläche der Untereinheit(en) (X) und ggf. zumindest ein Teil der freien Oberfläche der ggf. vorhandenen Trennschicht(en) (Z) mit wenigstens einer die Freisetzung der Komponente (c) und/oder (e) und/oder (d) und/oder (f) verhindernden Barriereschicht (Z') überzogen sein. Auch die Barriereschicht (Z') muss die erfindungsgemäßen Härtevoraussetzungen erfüllen.

Ebenfalls besonders bevorzugt ist eine Ausführungsform der erfindungsgemäßen Darreichungsform, die eine vertikale oder horizontale Anordnung der Schichten der Untereinheiten (X) und (Y) und wenigstens eine dazwischen angeordnete Push-Schicht (p) sowie ggf. eine Trennschicht (Z) aufweist, in der sämtliche freie Oberflächen des aus den Untereinheiten (X) und (Y), der Push-Schicht und der ggf. vorhandenen Trennschicht (Z) bestehenden Schichtaufbaus mit einem semipermeablen Überzug (E) ausgerüstet sind, der für ein Freisetzungsmedium, d.h. üblicherweise eine physiologische Flüssigkeit, durchlässig, für den Wirkstoff und für die Komponente (c) und/oder (d) und/oder (f) im wesentlichen undurchlässig ist, und wobei dieser Überzug (E) im Bereich der Untereinheit (X) wenigstens eine Öffnung zur Freisetzung des Wirkstoffes aufweist.

Eine entsprechende Darreichungsform ist dem Fachmann beispielsweise unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt.

In einer weiteren bevorzugten Ausführungsform hat die Untereinheit (X) der erfindungsgemäßen Darreichungsform die Form einer Tablette, deren Steg und ggf. eine der beiden Grundflächen mit einer die Komponente (c) und/oder (d) und/oder (f) enthaltenden Barriereschicht (Z') bedeckt ist.

Der Fachmann versteht, dass die bei der Formulierung der erfindungsgemäßen Darreichungsform jeweils zum Einsatz kommenden Hilfsstoffe der Untereinheit(en) (X) bzw. (Y) sowie ggf. der vorhandenen Trennschicht(en) (Z) und/oder der Barriereschicht(en) (Z') in Abhängigkeit von deren Anordnung in der erfindungsgemäßen Darreichungsform, der Applikationsart sowie in Abhängigkeit von dem jeweiligen Wirkstoff der ggf. vorhandenen Komponenten (a) und/oder (b) und/oder (e) und der Komponente (c) und/oder (d) und/oder (f) variieren. Die Materialien, die über die jeweils erforderlichen Eigenschaften verfügen sind, dem Fachmann an sich bekannt.

Sofern die Freisetzung der Komponente (c) und/oder (d) und/oder (f) aus der Untereinheit (Y) der erfindungsgemäßen Darreichungsform mit Hilfe einer Umhüllung, vorzugsweise einer Barriereschicht, verhindert wird, kann die Untereinheit aus üblichen, dem Fachmann bekannten Materialien bestehen, sofern sie wenigstens ein Polymer (C) und gegebenenfalls (D) zur Erfüllung der Härtebedingung der erfindungsgemäßen Darreichungsform enthält.

Ist eine entsprechende Barriereschicht (Z') zur Verhinderung der Freisetzung der Komponente (c) und/oder (d) und/oder (f) nicht vorgesehen, sind die Materialien der Untereinheiten so zu wählen, dass eine Freisetzung der jeweiligen Komponente (c) und/oder (d) aus der Untereinheit (Y) praktisch ausgeschlossen ist. Bevorzugt können hierzu die nachstehend aufgeführten Materialien zum Einsatz kommen, die auch für den Aufbau der Barriereschicht geeignet sind.

Bevorzugte Materialien sind solche, die ausgewählt sind aus der Gruppe umfassend Alkylcellulosen, Hydroxyalkylcellulosen, Glucanen, Skleroglucanen, Mannanen, Xanthanen, Copolymeren aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane sowie Polyurethane und deren Copolymeren.

Besonders geeignete Materialien können ausgewählt werden aus der Gruppe umfassend Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe umfassend Copolymere aus Butylmethacrylat und Isobutylmethacrylat, Copolymere aus Methylvinylether und Maleinsäure mit erhöhtem Molekulargewicht, Copolymere aus Methylvinylether und Maleinsäuremonoethylester, Copolymere aus Methylvinylether und Maleinsäureanhydrid sowie Copolymere aus Vinylalkohol und Vinylacetat.

Weitere, zur Formulierung der Barriereschicht besonders geeignete Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1).

Ggf. können die vorstehend genannten Materialien mit weiteren üblichen, dem Fachmann bekannten Hilfsstoffen, vorzugsweise ausgewählt aus der Gruppe umfassend Weichmacher, Gleitmittel, Antioxidantien, wie z. B. Glycerinmonostearat, halbsynthetische Triglyceridderivate, halbsynthetische Glyceride, hydriertes Rizinusöl, Glycerinpalmitostearat, Glycerinbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierte Triglyceride, Glyceride, Polyoxyalkylenglykole, Polyalkylenglykole und deren Derivate abgemischt werden.

Sofern die erfindungsgemäße Darreichungsform eine Trennschicht (Z') aufweist, kann diese, ebenso wie die nicht umhüllte Untereinheit (Y) vorzugsweise aus den vorstehend, für die Barriereschicht beschriebenen Materialien bestehen. Der Fachmann versteht, daß auch über die Dicke der Trennschicht die Freisetzung des Wirkstoffes bzw. der Komponente (c) und/oder (d) aus der jeweiligen Untereinheit gesteuert werden kann.

Die erfindungsgemäße Darreichungsform weist eine kontrollierte Freisetzung des Wirkstoffes auf. Sie eignet sich dabei vorzugsweise für eine 2x tägliche Verabreichung an Patienten.

Die erfindungsgemäße Darreichungsform kann einen oder mehrere Wirkstoffe mit Missbrauchspotential zumindest teilweise in einer darüber hinaus retardierten Form aufweisen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge. Die Wirkstoffabgabe muss aber so gesteuert sein, daß die vorstehend genannten Bedingungen jeweils erfüllt sind, z.B. das bei bestimmungsgemäßer Applikation der Darreichungsform der Wirkstoff bzw. die Wirkstoffe praktisch komplett freigesetzt wird, bevor die ggf, vorhandenen Komponente (c) und/oder (d) eine beeinträchtigende Wirkung entfalten können. Außerdem darf durch die Zugabe von retardierenden Materialien keine Beeinträchtigung der notwendigen Härte erfolgen.

Die kontrollierte Freisetzung aus der erfindungsgemäßen Darreichungsform wird vorzugsweise durch Einbettung des Wirkstoffes in eine Matrix erzielt. Die als Matrixmaterialien dienenden Hilfsstoffe kontrollieren die Wirkstofffreisetzung. Matrixmaterialien können beispielweise hydrophile, gelbildende Materialien sein, woraus die Wirkstofffreisetzung hauptsächlich durch Diffusion erfolgt, oder hydrophobe Materialien sein, woraus die Wirkstofffreisetzung hauptsächlich durch Diffusion aus den Poren in der Matrix erfolgt.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophoben Polymeren, Wachsen, Fetten, langkettigen Fettsäuren, Fettalkoholen oder entsprechenden Estern oder Ethem oder deren Gemische. Besonders bevorzugt werden als hydrophobe Materialien Mono- oder Digliceride von C12-C30-Fettsäuren und/oder C12-C30-Fettalkohole und/oder Wachse oder deren Gemische eingesetzt.

Es ist auch möglich, Mischungen der vorstehend genannten hydrophilen und hydrophoben Materialien als Matrixmaterialien einzusetzen.

Des weiteren können auch die Komponenten (C) und ggf. vorhandene Komponente (D), die zur Erzielung der erfindungsgemäß notwendigen Bruchfestigkeit von mindestens 500 N dienen, bereits als zusätzliche Matrixmaterialien dienen.

Sofern die erfindungsgemäße Darreichungsform zur oralen Applikation vorgesehen ist, kann sie bevorzugt auch einen magensaftresistenten Überzug aufweisen, der sich in Abhängigkeit vom pH-Wert der Freisetzungsumgebung auflöst. Durch diesen Überzug kann erreicht werden, daß die erfindungsgemäße Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf.

Entsprechende Materialien und Verfahren zur Retardierung von Wirkstoffen sowie zum Aufbringen magensaftresistenter Überzüge sind dem Fachmann beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt.

### Methode zur Bestimmung der Bruchfestigkeit

Zur Überprüfung, ob ein Material als Komponente (C) oder (D) eingesetzt werden kann, wird das Material zu einer Tablette mit einem Durchmesser von 10 mm und einer Höhe von 5mm mit einer Kraft von 150 N, bei einer Temperatur entsprechend mindestens dem Erweichungspunkt des Polymeren und bestimmt mit Hilfe eines DSC-Diagramms des Materials verpresst. Mit so hergestellten Tabletten wird gemäß der Methode zur Bestimmung der Bruchfestigkeit von Tabletten, veröffentlicht im Europäischen Arzneibuch 1997, Seite 143, 144, Methode Nr. 2.9.8. unter Einsatz der nachstehend aufgeführten Apparatur die Bruchfestigkeit bestimmt. Als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

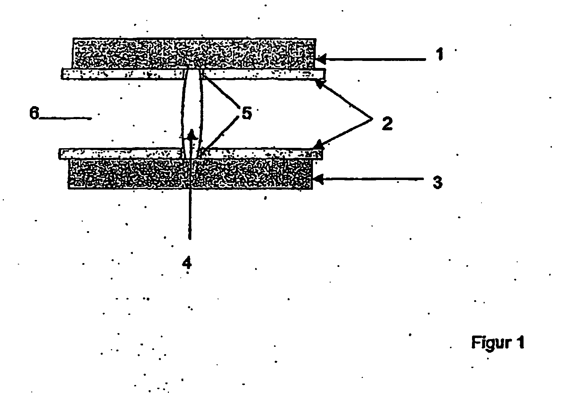

Figur 1 zeigt die Messung der Bruchfestigkeit einer Tablette, insbesondere die dafür eingesetzte Justierungsvorrichtung (6) der Tablette (4) vor und während der Messung. Darzu wird die Tablette (4) zwischen der oberen Druckplatte (1) und der unteren Druckplatte (3) der nicht dargestellten Vorrichtung zur Kraftaufbringung mit Hilfe von zwei 2-teiligen Einspannvorrichtungen, die jeweils mit der oberen bzw. unteren Druckplatte nach Einstellung des zur Aufnahme und zur Zentrierung der zu messenden Tablette notwendigen Abstands (5) fest verbunden (nicht dargestellt) werden. Zur Einstellung des Abstands (5) können die 2-teiligen Einspannvonichtungen jeweils auf der Druckplatte, auf der sie gelagert sind, horizontal nach außen oder innen bewegt werden.

Als bruchfest bei einer bestimmten Krafteinwirkung werden auch die Tabletten eingestuft, bei denen kein Bruch feststellbar, aber ggf. eine plastische Verformung der Tablette durch die Krafteinwirkung erfolgt ist.

Bei den erfindungsgemäß erhaltenen Darreichungsformen wird die Bruchfestigkeit nach der aufgeführten Meßmethode bestimmt, wobei von Tabletten abweichenden Darreichungsformen ebenso geprüft werden.

Im Folgenden wird die Erfindung anhand von Beispielen erläutert, Diese Erläuterungen sind lediglich beispielhaft und schränken den allgemeinen Erfindungsgedanken nicht ein.

### Beispiel 1:

| Komponenten | Pro Tablette | Gesamtansatz |
|---|---|---|
| Tramadolhydrochlorid | 100,0 mg | 1495,0 g |
| Polyethylenoxid, NF, MG 7 000 000 (Polyox WSR 303, Dow Chemicals) | 167,8 mg | 2508,6 g |
| Hydroxypropylmethyl- cellulose 100 000 m Pas | 33,5 mg | 500,8 g |
| Polyethylenglykol (PEG 6000) | 33,5 mg | 500,8 g |
| Butylhydroxytoluol (BHT) | 0,2 mg | 3,0 g |
| Gesamtgewicht | 335,0 mg | 5008,2 g |

Die angegebene BHT-Menge wurde in Ethanol (96 %) gelöst, so dass man eine 7,7%-ige (m/m) ethanolische Lösung erhielt. Diese wurde zunächst mit 150 g Polyethylenoxid in einem Schnellmischer für 30 Minuten gemischt und dann die restliche Mange Polyethylenoxid hinzu gegeben und erneut 30 Minuten gerührt. Die Masse wurde bei 40 °C 12 h getrocknet. Alle weiteren Komponenten wurden zugesetzt und in einem Freifallmischer 15 Minuten lang gemischt. Die Pulvermischung wurde in einen Extruder dosiert. Zur Extrusion wurde ein Doppelschneckenextruder der Fa. Leistritz (Nümberg) vom Typ Micro 27 GL 40 D, Spindeldurchmesser 18 mm, eingesetzt. Es wurden Schnecken mit stumpfen Schneckenenden eingesetzt, wobei der Innensechskant am Ende der Schnecken mit einer Kappe verschlossen war. Als Düse dient eine beheizbare Runddüse mit einem Durchmesser von 8 mm. Das gesamte Verfahren wurde unter einer N₂-Atmosphäre durchgeführt.

Zur Extrusion wurden folgende Parameter gewählt:

| | |
|---|---|
| Schneckendrehzahl: | 100 Upm |
| Durchsatz: | 4 kg/h |
| Produkttemperatur: | 125 °c |
| Manteltemperatur | 120 °C |

Das noch heiße Extrudat wurde unter einer Stickstoffatmosphäre abgekühlt. Der abgekühlte Strang wurde zu biplanen Tabletten vereinzelt.

Die Bruchfestigkeit der Tabletten wurde nach der angegebenen Methode bestimmt. Bei einer Krafteinwirkung von 500 N trat kein Bruch auf. Die Tabletten konnten weder mit einem Hammer noch mit Hilfe von Mörser und Pistill zerkleinert werden.

Die Färbung des abgekühlten Stranges bzw. der daraus vereinzelten 10 Tabletten wurden mit Hilfe von MUNSELL BOOK OF COLOUR mit N 9,5/ bestimmt, so dass die nach dem erfindungsgemäßen Verfahren hergestellte Darreichungsform keine Verfärbung durch die Thermoverformung mit Hilfe eines Extruders aufwies.

## Patentansprüche

1. Gegen Missbrauch gesicherte, ohne Verfärbung durch Extrusion thermogeformte Darreichungsform, **dadurch gekennzeichnet, dass** sie neben einem oder mehreren Wirkstoffen mit Missbrauchspotential (A) ausgewählt aus der Gruppe der Opioide, sowie ggf. physiologisch verträglichen Hilfsstoffen (B), mindestens ein Polyethylenoxid (C) mit einem Molekulargewicht von mindestens 0,5 Mio. aufweist.

2. Darreichungsform gemäß Anspruch 1, **dadurch gekennzeichnet, dass** sie in Form einer Tablette vorliegt.

3. Darreichungsform gemäß Anspruch 1, **dadurch gekennzeichnet, dass** sie in multipartikulärer Form vorliegt.

4. Darreichungsform gemäß Anspruch 3, **dadurch gekennzeichnet, dass** sie in Form von Mikrotabletten, Mikropellets, Granulaten, Sphäroiden, Perlen oder Pellets, ggf. zu Tabletten verpresst oder in Kapseln abgefüllt, vorliegt.

5. Darreichungsform gemäß einem der Ansprüche 1 bis 4, **dadurch gekennzeichnet, dass** das Molekulargewicht des Polyethylenoxids (C) mindestens 1 Mio. beträgt.

6. Darreichungsform gemäß Anspruch 5, **dadurch gekennzeichnet, dass** das Molekulargewicht des Polyethylenoxids (C) 1-15 Mio. beträgt.

7. Darreichungsform gemäß einem der Ansprüche 1 bis 6, **dadurch gekennzeichnet, dass** das Polyethylenoxid (C) in solchen Mengen vorliegt, dass die Darreichungsform eine Bruchfestigkeit von mindestens 500 N aufweist.

8. Darreichungsform gemäß einem der Ansprüche 1 bis 7, **dadurch gekennzeichnet, dass** sie noch mindestens eine der nachfolgenden Komponenten a)-f) aufweist:
(a) wenigstens einen den Nasen- und/oder Rachenraum reizenden Stoff;
(b) wenigstens ein viskositätserhöhendes Mittel, das in einem mit Hilfe einer notwendigen Mindestmenge an einer wässrigen Flüssigkeit aus der Darreichungsform gewonnenen Extrakt ein Gel bildet, welches vorzugsweise beim Einbringen in eine weitere Menge einer wässrigen Flüssigkeit visuell unterscheidbar bleibt;
(c) wenigstens einen Antagonisten für den Wirkstoff bzw. die Wirkstoffe mit Missbrauchspotential;
(d) wenigstens ein Emetikum;
(e) wenigstens einen Farbstoff als aversives Mittel;
(f) wenigstens einen Bitterstoff.

9. Darreichungsform gemäß Anspruch 8, **dadurch gekennzeichnet, dass** die Komponente (c) wenigstens ein Opioid-Antagonist ausgewählt aus der Gruppe bestehend aus Naloxon, Naltrexon, Nalmefen, Nalid, Nalmexon, Nalorphin, Nalbuphin und eine entsprechende physiologisch verträgliche Verbindung, insbesondere eine Base, ein Salz oder Solvat ist.

10. Darreichungsform gemäß einem der Ansprüche 1 bis 9, **dadurch gekennzeichnet, dass** sie wenigstens einen Werkstoff zumindest teilweise in retardierter Form enthält.

11. Darreichungsform gemäß Anspruch 10, **dadurch gekennzeichnet, dass** jeder der Wirkstoffe mit Missbrauchspotential (A) in einer Retardmatrix vorliegt.

12. Darreichungsform gemäß Anspruch 11, **dadurch gekennzeichnet, dass** das Polyethylenoxid (C) als Retardmatrixmaterial dient.

13. Verfahren zur Herstellung einer Darreichungsform gemäß einem der Ansprüche 1 bis 12, **dadurch gekennzeichnet, dass** man
z) die Komponenten (A), (B), (C) mischt sowie die ggf. vorhandenen Komponenten (a) bis (f) mitmischt oder, soweit notwendig, separat unter Zusatz des Polyethylenoxids (C) mischt,
y) die resultierende Mischung oder die resultierenden Mischungen im Extruder mindestens bis zum Erweichungspunkt des Polyethylenoxids (C) erwärmt und unter Krafteinwirkung durch die Austrittsöffnung eines Extruder extrudiert,
x) das noch plastische Extrudat vereinzelt und zur Darreichungsform formt oder
w) das abgekühlte und ggf. wieder erwärmte, vereinzelte Extrudat zur Darreichungsform formt,
wobei die Verfahrensschritte y) und x) und ggf. die Verfahrensschritte z) und w) unter Inertgasatmosphäre durchgeführt werden.

14. Verfahren gemäß Anspruch 13, **dadurch gekennzeichnet, dass** das vereinzelbare Extrudat pelletiert oder zu Tabletten gepresst wird.

15. Darreichungsform gemäß einem der Ansprüche 1 bis 12 erhältlich nach Verfahren gemäß einem der Ansprüche 13 bis 14.

## Claims

1. An abuse-proofed dosage form thermoformed by extrusion without discoloration, **characterised in that** in addition to one or more active ingredients with abuse potential (A) selected from the group of opioids and optionally physiologically acceptable auxiliary substances (B), it comprises at least one polyethylene oxide (C) with a molecular weight of at least 0.5 million.

2. A dosage form according to claim 1, **characterised in that** it is in the form of a tablet.

3. A dosage form according to claim 1, **characterised in that** it is in multiparticulate form.

4. A dosage form according to claim 3, **characterised in that** it is in the form of microtablets, micropellets, granules, spheroids, beads or pellets, optionally pressed into tablets or filled into capsules.

5. A dosage according to one of claims 1 to 4, **characterised in that** the molecular weight of the polyethylene oxide (C) is at least 1 million.

6. A dosage form according to claim 5, **characterised in that** the molecular weight of the polyethylene oxide (C) is 1-15 million.

7. A dosage form according to one of claims 1 to 6, **characterised in that** the polyethylene oxide (C) is present in such quantities that the dosage form exhibits a breaking strength of at least 500 N.

8. A dosage form according to one of claims 1 to 7, **characterised in that** it additionally comprises at least one of the following components a)-f):
(a) at least one substance which irritates the nasal passages and/or pharynx,
(b) at least one viscosity-increasing agent, which, with the assistance of a necessary minimum quantity of an aqueous liquid, forms a gel with the extract obtained from the dosage form, which gel preferably remains visually distinguishable when introduced into a further quantity of an aqueous liquid,
(c) at least one antagonist for the active ingredient or active ingredients with abuse potential,
(d) at least one emetic,
(e) at least one dye as an aversive agent,
(f) at least one bitter substance.

9. A dosage form according to claim 8, **characterised in that** component (c) is at least one opioid antagonist selected from the group comprising naloxone, naltrexone, nalmefene, nalide, nalmexone, nalorphine, nalbuphine and a corresponding physiologically acceptable compound, in particular a base, a salt or solvate.

10. A dosage form according to one of claims 1 to 9, **characterised in that** it contains at least one active ingredient at least partially in controlled release form.

11. A dosage form according to claim 10, **characterised in that** each of the active ingredients with abuse potential (A) is present in a controlled release matrix.

12. A dosage form according to claim 11, **characterised in that** the polyethylene oxide (C) serves as a controlled release matrix material.

13. A process for the production of a dosage form according to one of claims 1 to 12, **characterised in that**,
z) components (A), (B), (C) are mixed and the optionally present components (a) to (f) are co-mixed or, if necessary, are mixed separately with the addition of the polyethylene oxide (C),
y) the resultant mixture or the resultant mixtures is/are heated in the extruder at least up to the softening point of the polyethylene oxide (C) and extruded through the outlet orifice of an extruder by application of force,
x) the still plastic extrudate is singulated and formed into the dosage form or
w) the cooled and optionally reheated singulated extrudate is formed into the dosage form,
wherein process steps y) and x) and optionally process steps z) and w) are performed under an inert gas atmosphere.

14. A process according to claim 13, **characterised in that** the singulated extrudate is pelletised or pressed into tablets.

15. A dosage form according to one of claims 1 to 12 obtainable by a process according to one of claims 13 to 14.

## Revendications

1. Forme galénique protégée contre un usage détourné thermoformée par extrusion sans altération de couleur, **caractérisée en ce qu'**en plus d'une ou plusieurs substances actives à potentiel d'usage détourné (A), choisies dans le groupe des opioïdes, ainsi qu'éventuellement d'adjuvants (B) physiologiquement acceptables, elle comporte au moins un polyoxyéthylène (C) ayant une masse moléculaire d'au moins 0,5 million.

2. Forme galénique selon la revendication 1, **caractérisée en ce qu'**elle se trouve sous forme d'un comprimé.

3. Forme galénique selon la revendication 1, **caractérisée en ce qu'**elle se trouve sous forme multiparticulaire.

4. Forme galénique selon la revendication 3, **caractérisée en ce qu'**elle se trouve sous forme de microcomprimés, microgranules, granulés, sphéroïdes, billes ou granules, éventuellement pressés en comprimés ou introduits dans des gélules.

5. Forme galénique selon l'une quelconque des revendications 1 à 4, **caractérisée en ce que** la masse moléculaire du polyoxyéthylène (C) est d'au moins 1 million.

6. Forme galénique selon la revendication 5, **caractérisée en ce que** la masse moléculaire du polyoxyéthylène (C) vaut 1-15 millions.

7. Forme galénique selon l'une quelconque des revendications 1 à 6, **caractérisée en ce que** le polyoxyéthylène (C) est présent en quantités telles que la forme galénique présente une résistance à la rupture d'au moins 500 N.

8. Forme galénique selon l'une quelconque des revendications 1 à 7, **caractérisée en ce qu'**elle comporte encore au moins l'un des composants a) à f) suivants :
(a) au moins une substance irritant la cavité nasale et/ou pharyngienne ;
(b) au moins un agent augmentant la viscosité, qui, dans un extrait obtenu à partir de la forme galénique à l'aide d'une quantité minimale requise d'un liquide aqueux, forme un gel qui de préférence reste visuellement discernable lors de l'introduction dans une nouvelle quantité d'un liquide aqueux ;
(c) au moins un antagoniste de la substance active ou des substances actives à potentiel d'usage détourné ;
(d) au moins un vomitif ;
(e) au moins un colorant en tant qu'agent répulsif ;
(f) au moins une substance amère.

9. Forme galénique selon la revendication 8, **caractérisée en ce que** le composant (c) est au moins un antagoniste d'opioïde choisi dans le groupe constitué par la naloxone, la naltrexone, le nalmefène, le nalid, la nalmexone, la nalorphine, la nalbuphine et un composé physiologiquement acceptable correspondant, en particulier une base, un sel ou un produit de solvatation.

10. Forme galénique selon l'une quelconque des revendications 1 à 9, **caractérisée en ce qu'**elle contient au moins une substance active au moins en partie sous forme retard.

11. Forme galénique selon la revendication 10, **caractérisée en ce que** chacune des substances actives à potentiel d'usage détourné (A) se trouve dans une matrice retard.

12. Forme galénique selon la revendication 11, **caractérisée en ce que** le polyoxyéthylène (C) sert de matière de matrice retard.

13. Procédé pour la fabrication d'une forme galénique selon l'une quelconque des revendications 1 à 12, **caractérisé en ce que**
z) on mélange les composants (A), (B), (C) et on mélange avec ceux-ci les composants (a) à (f) éventuellement présents ou, si nécessaire, on les mélange séparément avec addition du polyoxyéthylène (C),
y) on chauffe le mélange résultant ou les mélanges résultants dans l'extrudeuse au moins jusqu'au point de ramollissement du polyoxyéthylène (C) et on l'extrude sous l'effet du froid par l'orifice de sortie d'une extrudeuse,
x) on isole l'extrudat encore plastique et on le moule en la forme galénique ou
w) on moule en la forme galénique l'extrudat isolé refroidi et éventuellement de nouveau chauffé,
en effectuant sous une atmosphère de gaz inerte les étapes y) et x) du procédé et éventuellement les étapes z) et w) du procédé.

14. Procédé selon la revendication 13, **caractérisé en ce qu'**on transforme en granules ou presse en comprimés l'extrudat isolable.

15. Forme galénique selon l'une quelconque des revendications 1 à 12, pouvant être obtenue selon l'une quelconque des revendications 13 et 14.
